(19) **Europäisches Patentamt / European Patent Office / Office européen des brevets**

(11) **EP 3 856 107 B1**

(12) **EUROPEAN PATENT SPECIFICATION**

(45) Date of publication and mention
of the grant of the patent:
**22.05.2024   Bulletin 2024/21**

(21) Application number: **19866386.6**

(22) Date of filing: **03.05.2019**

(51) International Patent Classification (IPC):
**A61F 13/15** *(2006.01)*      **A61F 13/49** *(2006.01)*

(52) Cooperative Patent Classification (CPC):
**A61F 13/15699; A61F 13/49012; A61F 13/49061**

(86) International application number:
**PCT/CN2019/085429**

(87) International publication number:
**WO 2020/062866 (02.04.2020 Gazette 2020/14)**

(54) **RING-LIKE ELASTIC BELT WITH IMPROVED SIDE SEAMS**

RINGARTIGES ELASTISCHES BAND MIT VERBESSERTEN SEITENNÄHTEN

CEINTURE ÉLASTIQUE ANNULAIRE DOTÉE DE COUTURES LATÉRALES AMÉLIORÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **25.09.2018   PCT/CN2018/107276**

(43) Date of publication of application:
**04.08.2021   Bulletin 2021/31**

(73) Proprietor: **The Procter & Gamble Company**
**Cincinnati, OH 45202 (US)**

(72) Inventors:
• **OGAWA, Kazuya**
**Akashi, Hyogo 6740093 (JP)**
• **LEE, Donsub**
**Akashi, Hyogo 6740093 (JP)**
• **SHIOMITSU, Daisuke**
**Akashi, Hyogo 6740093 (JP)**
• **YAMASHITA, Fumitake**
**Akashi, Hyogo 6740093 (JP)**
• **ZENG, Huasen**
**Guangzhou, Guangdong 510555 (CN)**
• **MORIMOTO, Koichi**
**Beijing 101312 (CN)**
• **CORTES, Maricel Soriano**
**Beijing 101312 (CN)**
• **LONG, Michael Devin**
**Cincinnati, Ohio 45202 (US)**
• **ORDWAY, David Carlton**
**Middletown Ohio 45042 (US)**
• **HUANG, Gene Xiaoqing**
**Cincinnati, Ohio 45202 (US)**
• **TSUJI, Masayuki**
**Akashi, Hyogo 6740093 (JP)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 3 326 595      WO-A1-2018/118581**
**CN-A- 106 137 542      US-A1- 2010 063 468**

**Description**

FIELD OF THE INVENTION

The present invention relates to ring-like elastic belts having improved side seams, articles comprising thereof, and methods of making thereof.

BACKGROUND OF THE INVENTION

Infants and other incontinent individuals wear absorbent articles such as pant to receive and contain urine and other body exudates. Pull-on absorbent articles, or pant-type absorbent articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult.

Belt-type pant having a center chassis to cover the crotch region of the wearer and a separate elastic belt defining the waist opening and leg opening are known in the art, such as described in PCT Publication WO 2006/1771 8A or US2010063468.

Such belt-type pant articles have an elastic belt made of a laminate of nonwoven layers and elastic bodies sandwiched therebetween. These elastic belts may be economically made by overlaying and joining two of such laminates to make the front and back belt, then seaming the laminates to make side seams. Recently, various nonwoven materials are used to meet consumer needs, and some material may have lower process tolerance. For example, nonwoven material having favorable lofty tactile sense may have a lower tensile breaking point. When the laminate is made of 2 nonwoven layers, one of the layers may be folded over to avoid having sharp edges at the waist opening or the leg opening. Thus, the elastic belt may result in a region having the fold over provided in 3 layers, while another region provided in 2 layers, giving a variation of caliper and basis weight along the side seam. Still further, the elastic belt may be structured to have an ergonomic fit, thereby having different positioning of elastic bodies coming from the front and back belt. All of the above mentioned non-homogeneity of the side seam may make the side seaming process difficult to control, and/or result in side seams that have varied quality of seaming strength.

Based on the foregoing, there is a need to provide a ring-like elastic belt for the belt-type pant article which has side seams which tolerate normal usage conditions, while also being easy to open after use for removal. There is further a need for a reliable high speed method of making side seams for the belt-type pant article over a variety of conditions for making the elastic belt, and in an economical manner.

SUMMARY OF THE INVENTION

The present invention is directed to ring-like elastic belt (104) having a longitudinal direction and a lateral direction comprising:

a front belt (106) and a back belt (108), each of the front belt (106) and back belt (108) being an elastic laminate comprising a first substrate layer (162), a second substrate layer (164), and a plurality of elastic bodies (168) joined between the first substrate layer (162) and the second substrate layer (164), each of the front belt (106) and the back belt (108) further comprising a fold over (162FO) of at least one of the first substrate layer (162) and the second substrate layer (164) wherein the fold over (162FO) of the front belt (106) has a longitudinal dimension shorter than the front belt (106), and the fold over (162FO) of the back belt (108) has a longitudinal dimension shorter than the front belt (106),
a pair of side seams which join the lateral edges of the front belt (106) and the back belt (108) such that the front fold over (162FO) and the back fold over (162FO) cooperatively define a distal edge of the ring-like elastic belt (104), the side seam made by a hot air bond which at least partially melts material of the elastic laminate, the side seam having continuity of the melted material along the substantial entirety of its longitudinal dimension;
wherein the side seam has a Belt Minimum Peel Strength of at least about 6N/25mm, a Belt Maximum Peel Strength of no more than 18N/25mm, and a Top Bottom Difference of no more than 15%, according to the measurements herein.

The present invention is also directed to methods of making such ring-like elastic belt (104).

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

Figure 1 is a perspective view of a pant.
Figure 2 is a partially cut away plan view of the pant shown in Figure 1.
Figure 3A is a cross-sectional view of the pant of Figure 2 taken along line 3A-3A.
Figure 3B is a cross-sectional view of the pant of Figure 2 taken along line 3B-3B.
Figure 4 is a schematic side view of a converting apparatus adapted to manufacture a pant.
Figure 5A is a view of multiple discrete chassis spaced from each other along the machine direction and being connected to the continuous front and back belts, as taken along line A-A from Figure 4.
Figure 5B is a view of multiple discrete chassis and continuous front and back belts connected with each being folded, as taken along line B-B from Figure 4.
Figure 5C is a view of folded continuous articles, as taken along line C-C from Figure 4.
Figure 5D is a view of two discrete pant articles, as taken along line D-D from Figure 4.
Figure 6A1 is a schematic side view of a first embodiment of a bonder module.

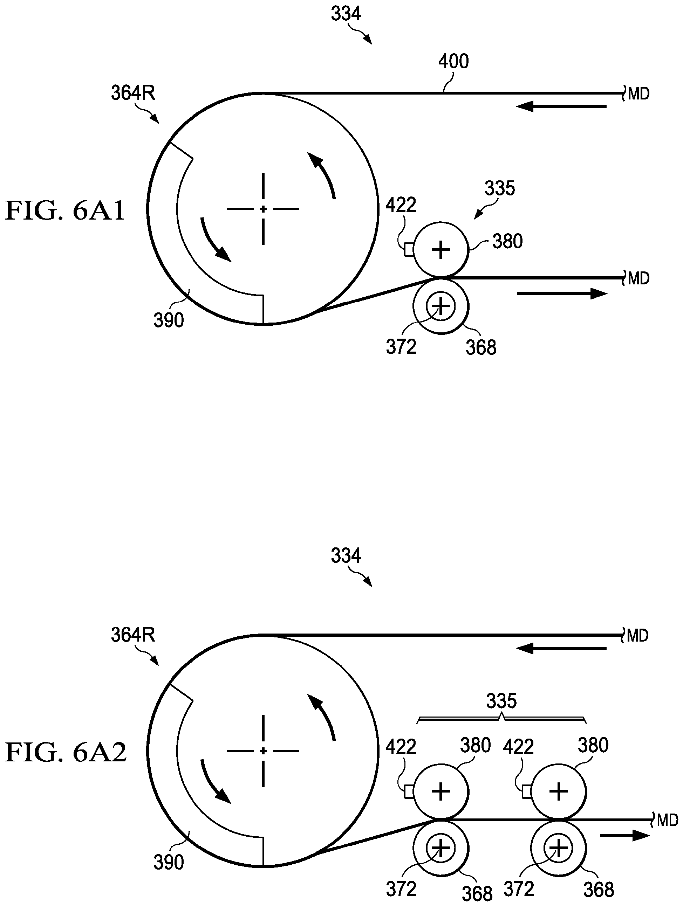

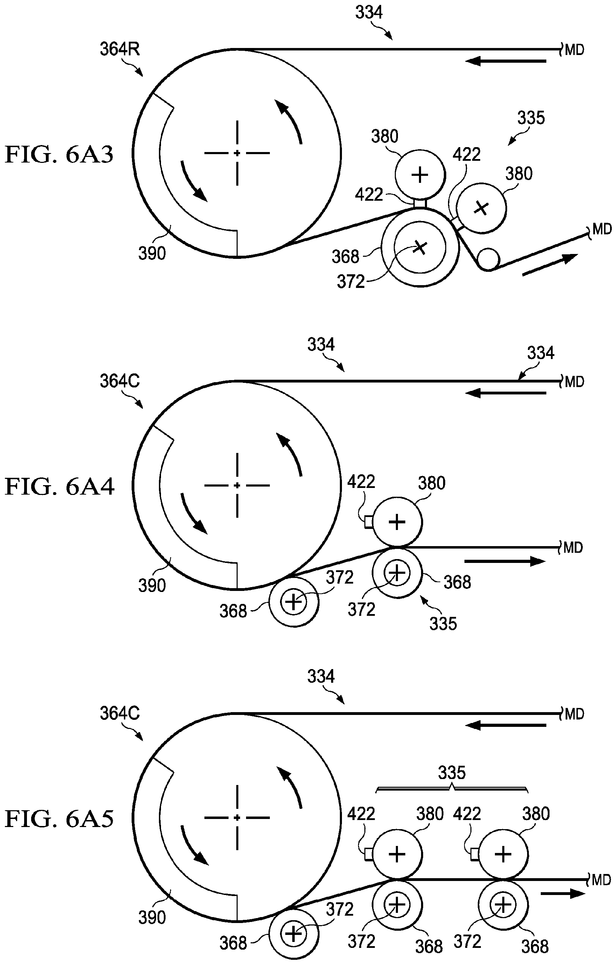

Figure 6A2 is a schematic side view of a second embodiment of a bonder module.
Figure 6A3 is a schematic side view of a third embodiment of a bonder module.
Figure 6A4 is a schematic side view of a fourth embodiment of a bonder module.
Figure 6A5 is a schematic side view of a fifth embodiment of a bonder module.

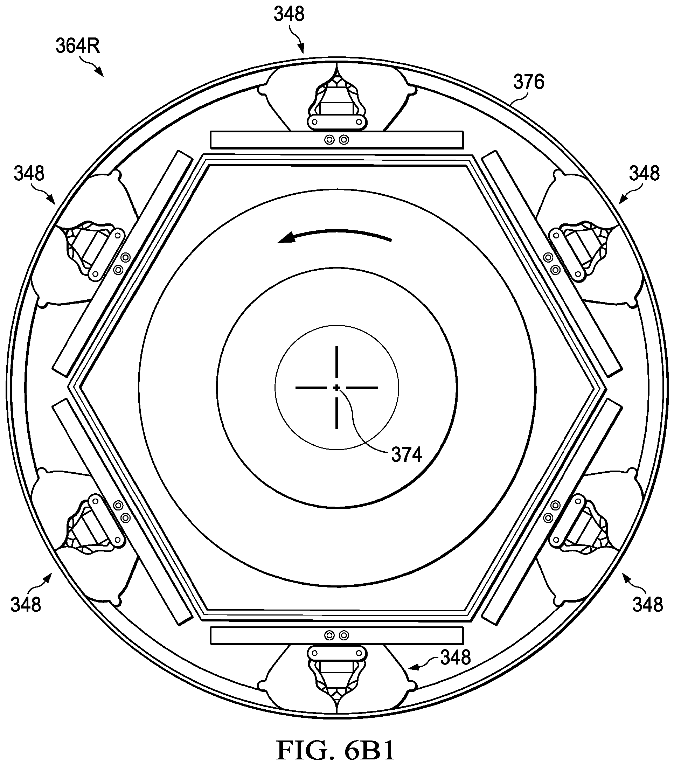

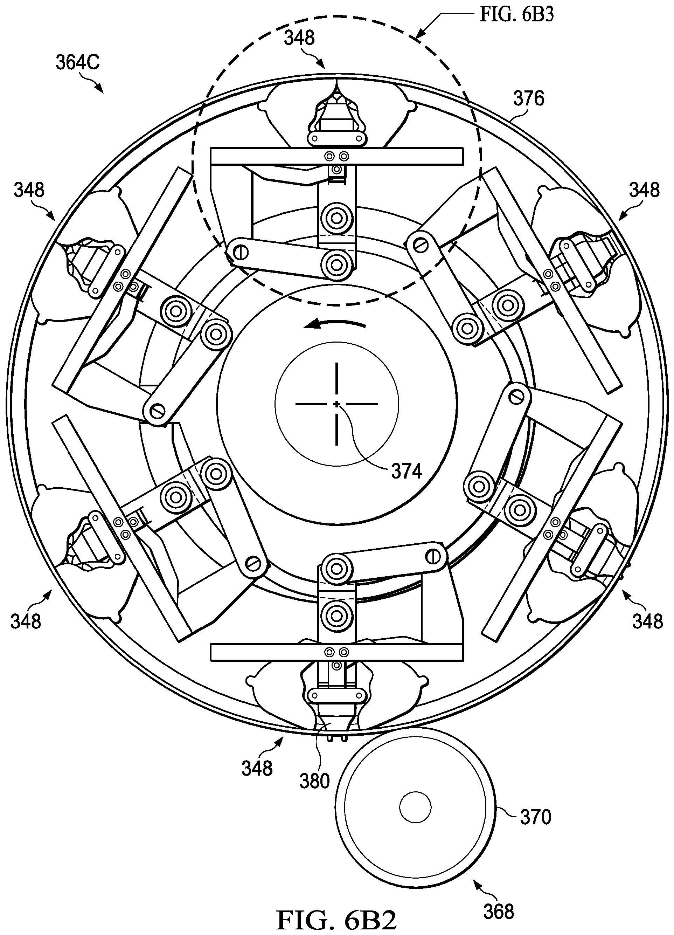

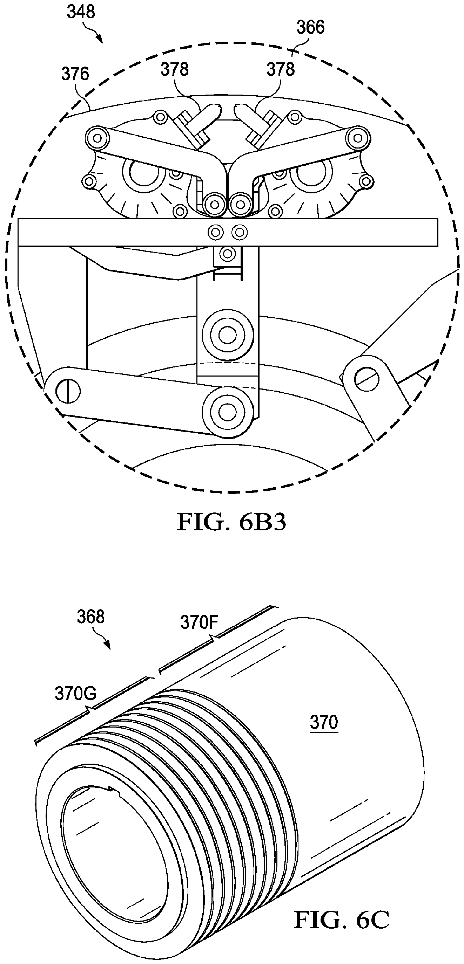

Figure 6B 1 is a schematic side view of a seaming drum.
Figure 6B2 is a schematic side view of another seaming drum.
Figure 6B3 is an elevation view of a seaming station of Figure 6B2 showing the hot air nozzles.
Figure 6C is an elevation view of an anvil roll.

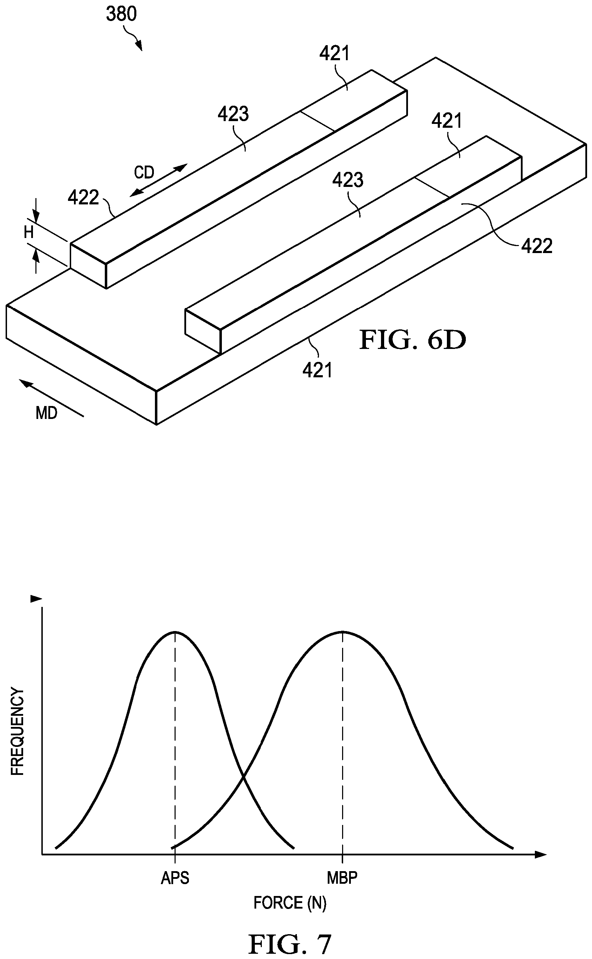

Figure 6D is a detailed, perspective view of a pressing member.
Figure 7 is a model of the force distribution charts for obtaining the Average Peel Strength and the Material Breaking Point, according to measurements herein.

DEFINITIONS

As used herein, the following terms shall have the meaning specified thereafter:
"Wearable article" refers to articles of wear which may be in the form of pant, taped pant, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.
"Pant" refers to wearable articles having a pre-formed waist and leg openings. A pant may be donned by inserting a wearer's legs into the leg openings and sliding the pant into position about the wearer's lower torso. Pant are also commonly referred to as "closed pant", "prefastened pant", "pull-on pant", "training pant" and "pant-pant".
"Longitudinal" refers to a direction running substantially perpendicular from a waist edge to an opposing waist edge of the article and generally parallel to the maximum linear dimension of the article. "Lateral" refers to a direction perpendicular to the longitudinal direction.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).
"Disposed" refers to an element being located in a particular place or position.
"Joined" refers to configurations whereby an element is directly secured to another element by affixing the element directly to the other element and to configurations whereby an element is indirectly secured to another element by affixing the element to intermediate member(s) which in turn are affixed to the other element.
"Proximal" refers to a portion being closer relative to the longitudinal center of the article, while "distal" refers to a portion being farther from the longitudinal center of the article.
"Water-permeable" and "water-impermeable" refer to the penetrability of materials in the context of the indented usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Radial" means a direction running from the center of a drum toward a drum outer circumferential surface.

"Substrate" means a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

"Nonwoven" means a material made from continuous (long) filaments (fibers) and/or discontinuous (short) filaments (fibers) by processes such as spunbonding, meltblowing, carding, and the like. Nonwovens do not have a woven or knitted filament pattern.

"Machine direction" means the direction of material flow through a process. In addition, relative placement and movement of material can be described as flowing in the machine direction through a process from upstream in the process to downstream in the process.

"Cross machine direction" means a direction that is generally perpendicular to the machine direction.

DETAILED DESCRIPTION OF THE INVENTION

Ring-like Elastic Belt

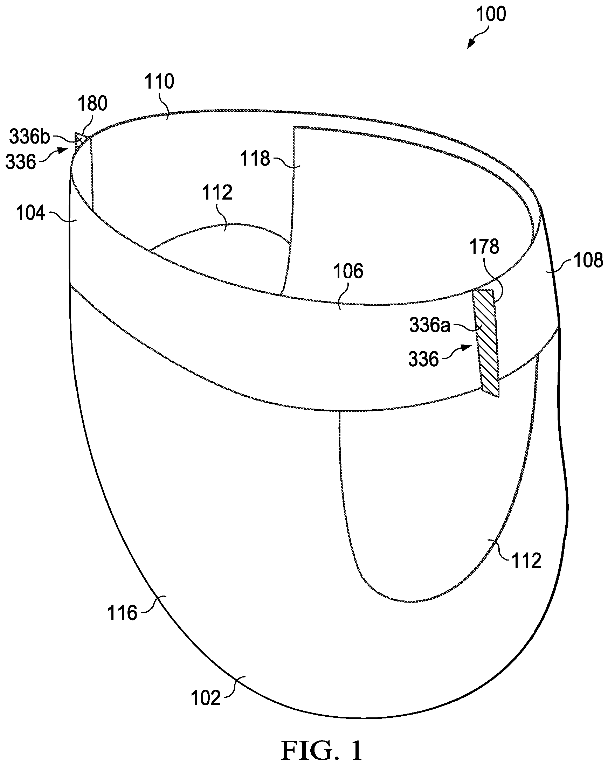

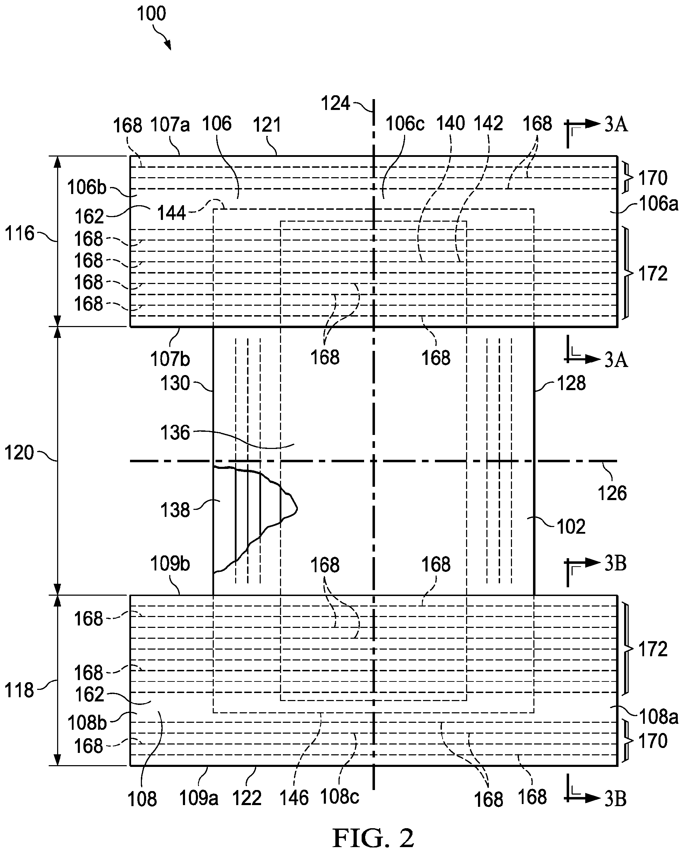

Figures 1 and 2 show an example of a belt-type pant (100) comprising the ring-like elastic belt (104) of the present invention. Figure 1 shows a perspective view of a belt-type pant (100) in a pre-fastened configuration, and Figure 2 shows a schematic plan view of the belt-type pant (100) with the seams unjoined and in a flat uncontracted condition showing the garment facing surface. The belt-type pant (100) shown in Figures 1 and 2 includes a chassis (102) and a ring-like elastic belt (104). As discussed below in more detail, a front belt (106) and a back belt (108) are bonded together to form the ring-like elastic belt (104).

With continued reference to Figure 2, the article includes a front waist region (116), a back waist region (118), and a crotch region (120) disposed intermediate the front and back waist regions. The pant (100) may also include a laterally extending distal edge (121) in the front waist region (116) and a longitudinally opposing and laterally extending distal edge (122) in the back waist region (118). To provide a frame of reference, the pant (100) and chassis (102) of Figure 2 are shown with a longitudinal axis (124) and a lateral axis (126). The longitudinal axis (124) may extend through the front waist edge (121) and through the back waist edge (122). And the lateral axis (126) may extend through a first longitudinal or right side edge (128) and through a midpoint of a second longitudinal or left side edge (130) of the article.

As shown in Figures 1 and 2, the belt-type pant (100) may comprise a chassis (102) including a backsheet (136) and a topsheet (138). The chassis (102) may also include an absorbent assembly (140), including an absorbent core (142), disposed between a portion of the topsheet (138) and the backsheet (136). The pant (100) may also include other features, such as leg elastic bodies (168) and/or leg cuffs to enhance the fit around the legs of the wearer.

As shown in Figure 2, the periphery of the chassis (102) may be defined by the first longitudinal side edge (128), a second longitudinal side edge (130), a first laterally extending end edge (144) disposed in the first waist region (116), and a second laterally extending end edge (146) disposed in the back waist region (118). Both side edges (128, 130) extend longitudinally between the first end edge (144) and the second end edge (146). As shown in Figure 2, the laterally extending end edges (144, 146) are located longitudinally inward from the laterally extending front waist edge (121) in the front waist region (116) and the laterally extending back waist edge (122) in the back waist region (118). When the belt-type pant (100) is worn on the lower torso of a wearer, the front distal edge (121) and the back distal edge (122) of the chassis (102) may encircle a portion of the waist of the wearer. At the same time, the chassis side edges (128, 130) may encircle at least a portion of the legs of the wearer. And the crotch region (120) may be generally positioned between the legs of the wearer extending from the front waist region (116) through the crotch region (120) to the back waist region (118).

The belt-type pant (100) comprising the ring-like elastic belt (104) of the present invention is provided to consumers in a configuration wherein the front waist region (116) and the back waist region (118) are connected to each other as packaged, prior to being applied to the wearer. As such, the belt-type pant (100) may have a continuous perimeter waist opening (110) and continuous perimeter leg openings (112) such as shown in Figure 1. The ring-like elastic belt (104) is defined by a front belt (106) connected with a back belt (108). As shown in Figure 2, the front belt (106) defines left and right side regions (106a, 106b) and a central region (106c), and the back belt (108) defines left and right side regions (108a, 108b) and a central region (108c).

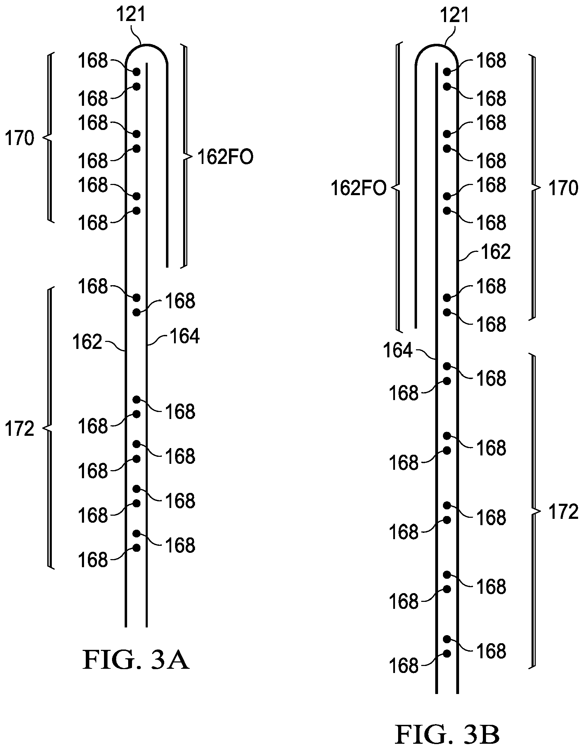

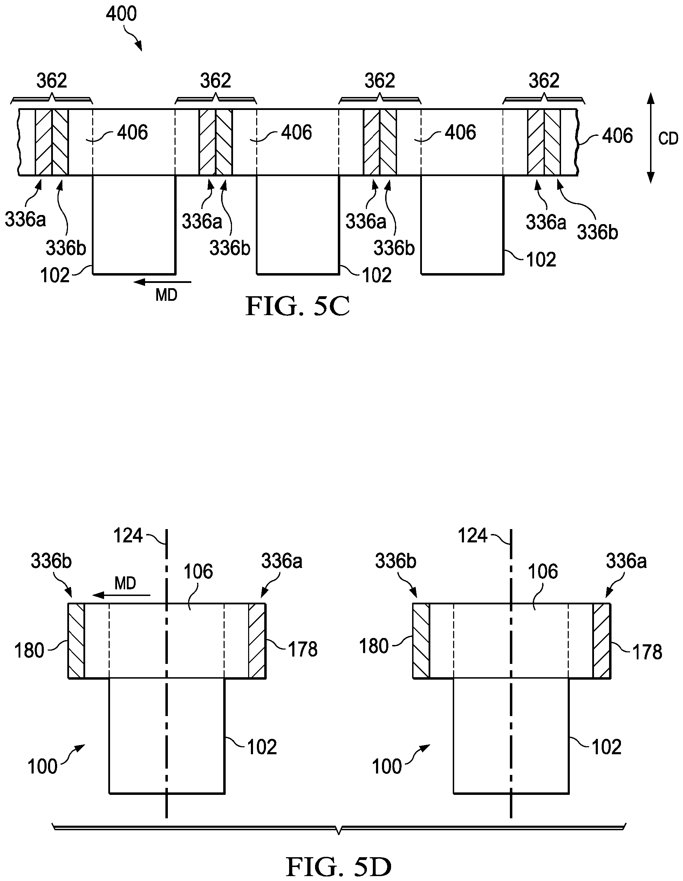

The central region (106c) of the first elastic belt may be connected with the front waist region (116) of the chassis (102), and the central region (108c) of the back belt (108) may be connected with the back waist region (118) of the chassis (102). As shown in Figure 1, the left side region (106a) of the front belt (106) is connected with the left side region (108a) of the back belt (108) at first side seam (178), and the right side region (106b) of the front belt (106) is connected with the right side region (108b) of the back belt (108) at second side seam (180) to define the ring-like elastic belt (104) as well as the waist opening (110) and leg openings (112). As shown in Figures 2, 3A, and 3B, the front belt (106) also defines a distal edge (107a) and a proximal edge (107b), and the back belt (108) defines a distal edge (109a) and a proximal edge (109b). The distal edges (107a, 107b) may also define the front waist edge (121) and the laterally extending back waist edge (122). Referring to Figure 1, when assembled as a pant article, the areas in which the side seams (178, 180) are formed do not overlap with the chassis (102) but have the body facing side of the front and back belt (106, 108) directly facing each other. Referring to Figure 5C, such area in which the front and back belt (106, 108) directly overlap with each other is defined as the overlap area (362).

Referring to Figures 2, 3A, and 3B, each of the front belt (106) and back belt (108) may impart elasticity by forming a laminate comprising a first substrate layer (162), a second substrate layer (164), and a plurality of elastic bodies (168) joined between the first substrate layer (162) and the second substrate layer (164). The plurality of elastic bodies (168) may be disposed to extend in the lateral direction. The plurality of elastic bodies (168) may be disposed to extend parallel with each other. The plurality of elastic bodies (168) may also be referred to herein as outer elastic bodies (170) and inner elastic bodies (172). The first substrate layer (162) may have a greater longitudinal dimension that the second substrate layer (164), and thus extend beyond the distal edge of the second substrate layer (164) to form a fold over (162FO) wherein the fold over (162FO) is folded at the distal edge and attached to the laminate. In Figures 3A and 3B, the fold over (162FO) is folded in a manner sandwiching the second substrate layer (164), however, the fold over (162FO) may also be folded away from the second substrate layer (164) and attached to the first substrate layer (162). The fold over (162FO) may avoid sharp distal edges, or provide loftiness to the elastic laminate. The fold over (162FO) of the front belt (106) has a longitudinal dimension shorter than the front belt (106), and the fold over (162FO) of the back belt (108) has a longitudinal dimension shorter than the front belt (106). Due to this fold over (162FO), the laminates are provided in regions having two substrate layers and three substrate layers. The inner elastic bodies (172) may be disposed in the two layer region, while the outer elastic bodies (170) may be disposed in the three layer region. The three layer region may have a maximum basis weight of at least about 90gsm.

The first and second substrate layers (162, 164) may be the same or different material, and selected to provide characteristics such as breathability, softness, cushiony feel, loftiness, and combinations thereof, depending on the desirables of the resulting article. The first and second substrate layers (162, 164) may have the same or different basis weight, stiffness, texture or any combination thereof. The first and second substrate layer layers (162, 164) may have a basis weight of from about 5gsm to about 45gsm, and may be made by processes such as meltblown, spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The first and second substrate layers (162, 164) may also be a multilayer or composite structure combining nonwovens made by different processes and fibers such as combining spunbond and carded nonwovens. The first and second substrate layers (162, 164) may be made by biodegradable material, or derived from renewable resources.

One or both of the first and second substrate layers (162, 164) may have a plurality of openings or bondings. While openings and bondings provide various benefits for the wearer such as loft, softness and breathability, such treatment may also provide the substrate layer to have a relatively low Material Breaking Point, according to the measurements herein, for example a Material Breaking Point of less than about 8N, or less than about 7N. When the substrate layer has a relatively low Material Breaking Point, this may lead to the substrate layer giving away when the side seam is torn to open the article after use. Namely, due to the force applied to the side seam to tear open the side seam in the lateral direction of the article, the substrate may rip in this direction. The ring-like elastic belt of the present invention has selected side seam properties to avoid such ripping, even when substrate layers of relatively low Material Breaking Point are used.

One or both of the first and second substrate layers (162, 164) may have a plurality of openings. The openings may have an opening rate of from about 5% to about 50%, and an effective opening area of from about $0.1mm^2$ to about $25mm^2$. The openings may be apertures, slits, or the like. The openings may be apertures having an aspect ratio of less than about 5. The openings may be made by female-male hot pin process, hole punching process, hydroentanglement process using water jets and a screen to create holes, and combinations thereof. The openings may be made by creating a plurality of weakened locations by heat or pressure followed by incremental stretching, causing said nonwoven web to rupture at the weakened locations such as described in US Patent 5,628,097, all hereby incorporated by reference. Such rupturing method may be particularly useful for nonwovens using spunbonded fibers and meltblown fibers. The openings may be three-dimensional, non-homogenous, unaligned and forming a pattern as disclosed in PCT Publication WO 2016/73712.

One or both of the first and second substrate layers (162, 164) may have a plurality of bondings. The bondings may have a bonding area of from about 4% to about 18%, and a bonding area of from about $0.3mm^2$ to about $10mm^2$. The bondings may be in various shapes, including but not limited to circles, ovals, straight or curved lines, and may be discrete or connected. Bondings may be referred to as embossings or quiltings. Bondings may be made by calendar bonding, optionally aided by air compression. The bondings may be those disclosed in PCT Publication WO 2012/134988.

Non-limiting examples of materials suitable for the first and second substrate layers (162, 164) of the present invention include: 12-30gsm air-through carded nonwoven substrate made of PE/PET bi-component staple fiber, such as those available from Beijing Dayuan Nonwoven Fabric Co. Ltd. or Xiamen Yanjan New Material Co. Ltd., and 8-30gsm spun melt nonwoven substrate comprising PP monofilament or PE/PP bi-component fibers, such as those available from Fibertex or Fitesa.

Referring to Figures 3A and 3B, the elastic bodies (168) may be disposed in the same or different denier, interval, or force between the front and back, as well as in different longitudinal positions of the belt. The elastic bodies (168) may be intermittently disposed in varying intervals, wherein the pattern of intervals of elastic bodies (168) disposed on the front belt (106) may be different from the pattern of intervals of the elastic bodies (168) disposed on the back belt (108). As such, the elastic bodies (168) may be disposed in a manner such that at least one elastic body from the front belt (106) and at least one elastic body from the back belt (108) overlap with each other at the side seam, while others do not. The elastic bodies (168) may be intentionally disposed to provide various benefits to the ring-like elastic belt (104), including ergonomic fit, anchoring against certain part of the wearer's body, accommodating movement of the legs, such as for example, the articles disclosed in WO2016/029651, WO2016/029652, WO2016/029653, WO2016/029566, WO2016/029655, WO2016/029656, WO2017/132852, WO2017/133031,

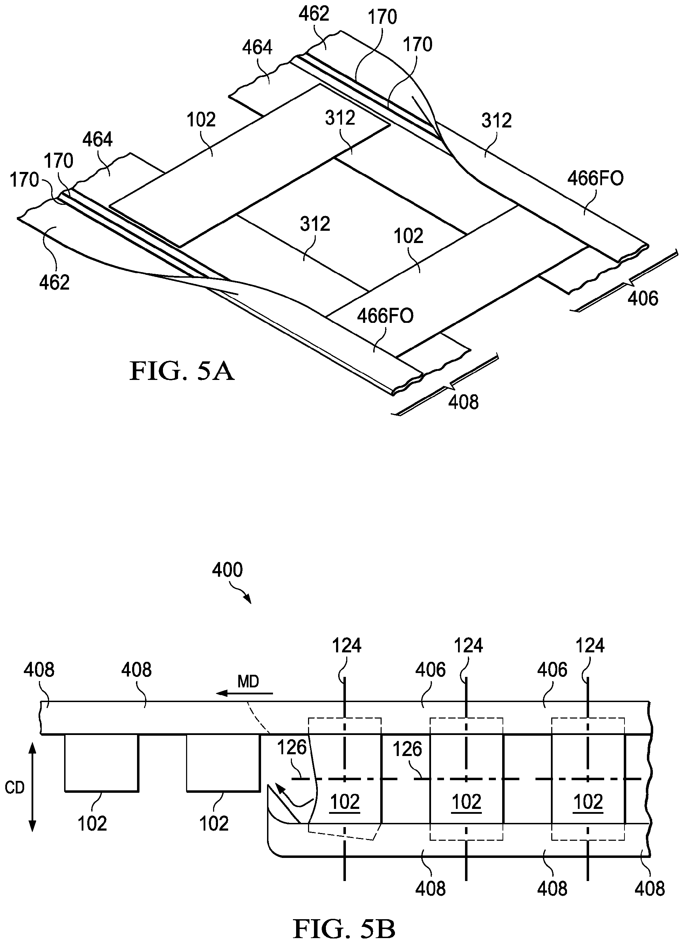

Still referring to Figures 3A and 3B, the longitudinal dimension of the front belt (106) may be different from the longitudinal dimension of the back belt (108), wherein either one of the longitudinal edges of the front belt (106) and back belt (108) may be matched with each other. The back belt (108) may have a greater longitudinal dimension wherein the distal edges of the front belt (106) and back belt (108) are matched to create a buttock cover on the back side. The front belt (106) may have a greater longitudinal dimension wherein the proximal edges of the front belt (106) and back belt (108) are matched to create a stomach cover on the front side. The longitudinal dimension of the fold over (162FO) of the front belt (106) may be different from the longitudinal dimension of the fold over (162FO) of the back belt (108). As described in further detail below and referring to Figure 5A, the fold over (162FO) may sandwich the chassis (102) to cover the longitudinal edges of the chassis (102) on the front and back. The chassis (102) may be placed offset from the longitudinal center of the article, namely shifted to the front side or the back side. For example, the chassis (102) may be shifted toward the front side to accommodate urine absorption. The front and back fold over (162FO) may be adjusted according to the position of the chassis (102) relative to the article.

Referring to Figure 1, the first and second side seams (178, 180) which join the lateral edges of the front belt (106) and the back belt (108) are made of material of the elastic laminate for forming the front belt (106) and the back belt (108). In the present invention, the side seams are made by a hot air bond which at least partially melts material of the elastic laminate forming the front belt (106) and the back belt (108), the side seams (178, 180) having continuity of the melted material along the substantial entirety of its longitudinal dimension. As discussed in further detail below, the side seams (178, 180) herein are formed by hot air bonding. Hot air bonding is advantageous in that the process may be conducted in high speed to form a reliable strong seam. It is desired that the side seam is strong enough to tolerate normal usage conditions, namely does not fail when stretched upon application, or after the article is loaded. On the other hand, it is also desired that the seam is easy to open after use, namely possible to tear by hand along the longitudinal dimension for removal from the wearer.

The side seam of the present invention has a Belt Minimum Peel Strength of at least about 6N/25mm, or at least about 8N/25mm, and a Belt Maximum Peel Strength of no more than about 18N/25mm, according to measurements herein. What is meant by Belt Minimum/Maximum Peel Strength is the average minimum/maximum peel strength among the 4 portions of the seam over a certain number of ring-like elastic belts. Specifically, the strength of a side seam may be represented by 4 (four) unique parts of the 2 (two) seams per ring-like elastic belt, namely the distal (top) edges and proximal (bottom) edges of the opposed longitudinal edges of the left and right seams. These four unique parts may be identified as "top left", "top right", "bottom left", and "bottom right", and their forces identified as codes FTL, FTR, FBL, and FBR. An average peel strength of each of the four unique parts may be obtained over a certain number of ring-like elastic belts. What is meant by Belt Minimum Peel Strength is the lowest peel strength among the four unique parts. What is meant by Belt Maximum Peel Strength is the highest peel strength among the four unique parts. By controlling the four unique parts to have a seam strength within the required values over a certain number of ring-like elastic belts, a ring-like elastic belt having a seam strength that endures premature tearing during use while also being easy to open after use may be stably manufactured.

The side seam of the present invention has a Top Bottom Difference of no more than about 15%, or no more than about 13%, according to measurements herein. Referring to the peel strengths among the 4 portions of the seam described above, the Top Bottom Difference is obtained as the absolute value of difference between the top forces FTL and FTR compared to the bottom forces FBL and FBR:

$$\left| \{(\mathrm{FTL} + \mathrm{FTR}) - (\mathrm{FBL} + \mathrm{FBR})\} \div (\mathrm{FBL} + \mathrm{FBR}) \right| \quad (\%)$$

When the Top Bottom Difference is controlled to a small deviation, the peeling experience from the top to bottom of the seam is perceived smooth and easy.

The side seam of the present invention may also have an Average Peel Strength, wherein the combined first and second substrate layer of the elastic belt has a Material Breaking Point according to measurements herein, wherein the Average Peel Strength is from about 20% to about 50% of the Material Breaking Point. By Average Peel Strength, what is meant is the greatest frequency force when a force distribution is recorded upon opening the side seam. By controlling the difference between the Average Peel Strength and Material Breaking Point as such, the elastic belt may be made of material having favorable tactile sense, while also having robust side seam strength.

The measurements for obtaining the Seam Maximum Peel Strength, the Seam Minimum Peel Strength, the Belt Maximum Peel Strength, the Belt Minimum Peel Strength, the Average Peel Strength and Material Breaking Point, are provided in further detail below.

Method of Manufacture

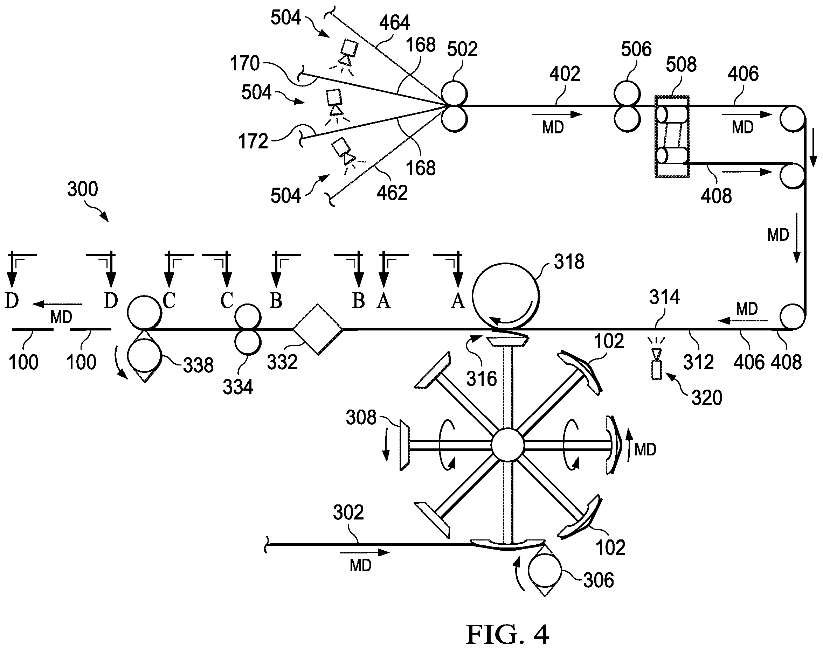

The present invention is also directed to methods of manufacturing the ring like elastic belt (104) described above, which may be assembled with a chassis (102) to make a belt-type pant article (100). For example, Figure 4 shows a schematic view of a converting apparatus (300) adapted to manufacture a pant (100). The method of operation of the converting apparatus (300) may be described with reference to the various components of pant (100) described above and shown in Figures 1 and 2. Although the following methods are provided in the context of the pant (100) shown in Figures 1 and 2, various types of pant (100) may be manufactured according to the methods disclosed herein, such as for example, the absorbent articles disclosed in; U.S. Patent Publication Nos. 2005/0107764 A1, 2012/0061016 A1, and 2012/0061015 A1.

The converting apparatus (300) shown in Figure 4 operates to advance discrete chassis (102) along a machine direction such that the lateral axis of each chassis (102) is parallel with the machine direction, and wherein the chassis (102) are spaced apart from each other along the machine direction. Referring to Figures 1 and 4, opposing waist regions (116, 118) of the spaced apart chassis (102) are then connected with continuous front and back belts (406, 408). The chassis (102) are then folded along the lateral axis to bring the continuous front and back belts (406, 408) into a facing relationship, and the continuous front and back belts (406, 408) are bonded together with bonds (336). As discussed in more detail below, the continuous front and back belts (406, 408) are bonded together with adjacent bonds (336a, 336b) intermittently spaced along the machine direction. Each bond (336a, 336b) may be a discrete bond site extending contiguously in the cross direction across a width of the continuous front and back belts (406, 408). The continuous front and back belts (406, 408) are then cut in the cross machine direction between the adjacent bonds (336a, 336b) to create discrete pant (100), such as shown in Figure 1.

As shown in Figure 4, a continuous first substrate layer (462), a continuous second substrate layer (464), and a plurality of elastic bodies (168) are combined to form a continuous elastic laminate (402). More particularly, the continuous first substrate layer (462) has a first surface and an opposing second surface and defines a width in a cross machine direction, the continuous second substrate layer (464) has a first surface and an opposing second surface and has a smaller width than the continuous first substrate layer (462), and the elastic bodies (168) are advanced in a machine direction and combined at nip rolls (502) to form a continuous elastic laminate (402), wherein the elastic bodies (168) are joined between the first surface of the continuous first substrate layer (462) and the first surface of the continuous second substrate layer (464). Before entering the nip rolls (502), the elastic bodies (168) are stretched in the machine direction. The elastic bodies may be categorized into outer elastic bodies (170) and inner elastic bodies (172). For joining the continuous first substrate layer (462), the continuous second substrate layer (464), and elastic bodies (170, 172), adhesive (504) may be applied to the elastic bodies (168) as well as either or both the continuous first substrate layer (462) and continuous second substrate layer (464) for joining the elastic bodies (170, 172) between the first surface of the continuous first substrate layer (462) and the first surface of an opposing continuous second substrate layer (464). Alternatively, the inner and outer elastic bodies (170, 172) may be disposed between the continuous first substrate layer (462) and the continuous second substrate layer (464), and sent to an ultrasonic joining station for joining with each other.

Referring to Figure 5A, the excess width of the continuous first substrate layer (462) may be folded such that the continuous first substrate layer (462) is folded along the machine direction of at least one of the continuous front belt (406) and the continuous back belt (408), the folded portion of the continuous first substrate layer defining a continuous fold over (466FO) region. The folding may be made towards the body facing side such that the first surface of the continuous first substrate layer (462) is joined against itself and/or the second surface of the continuous second substrate layer (464) as in Figures 3A, 3B, and 5A. Alternatively, the folding may be made towards the garment facing side such that the second surface of the continuous first substrate layer (462) is joined against itself (not shown).

Referring again to Figure 4, from the nip rolls (502) the continuous elastic laminate (402) advances in the machine direction to a cutter (506) that cuts the continuous elastic laminate (402) into two continuous belt substrates, referred to as a continuous front belt (406) and a continuous back belt (408). The cutter (506) may be configured in various ways. For example, in some embodiments the cutter (506) may be a slitter or a die cutter that separates the belt material into two continuous belt substrates with either a straight line cut and/or a curved line cut. The cutter (506) may also be configured as a perforator that perforates the belt material with a line of weakness and wherein the belt material is separated along the line of weakness in a later step. From the cutter (506), the continuous front and back belts (406, 408) advance through a diverter (508) that separates the continuous front and back belts (406, 408) from each other in the cross machine direction. The continuous front and back belts (406, 408) advance from the diverter (508) to a nip (316) between the carrier apparatus (308) and a roll (318).

As shown in Figure 4, a continuous length of chassis assemblies (302) are advanced in a machine direction to a carrier apparatus (308) and cut into discrete chassis (102) with knife roll (306). After the discrete chassis (102) are cut by the knife roll (306), the carrier apparatus (308) rotates and advances the discrete chassis (102) in the machine direction, wherein the longitudinal axis (124) of the chassis (102) is generally parallel with the machine direction. The carrier apparatus (308) also rotates while at the same time changing the orientation of the advancing chassis (102). The carrier apparatus (308) may also change the speed at which the chassis (102) advances in the machine direction. Various forms of carrier apparatuses may be used with the methods herein, such as for example, the carrier apparatuses disclosed in U.S. Patent No. 7,587,966 and U.S Patent Application Nos. 13/447,585; 13/447,568; 13/447,544; and 13/447,531.

As discussed below with reference to Figures 4, 5A, 5B, 5C and 5D, the chassis (102) are transferred from the carrier apparatus (308) and combined with advancing continuous front and back belts (406, 408), which are subsequently cut to form front and back belts (106, 108) to form the finished article (100).

With reference to Figure 4, the chassis (102) are transferred from the carrier apparatus (308) to a nip (316) between the carrier apparatus (308) and a roll (318) where the chassis (102) is combined with continuous front and back belts (406, 408). The body facing surface (312) of the continuous front belt (406) may be combined with the garment facing surface (314) of the chassis (102) along the first waist region (116), and the body facing surface (312) of the continuous back belt (408) may be combined with the garment facing surface (314) of the chassis (102) along the second waist region (118). As shown in Figure 4, adhesive (320) may be intermittently applied to the body facing surface (312) of the continuous front and back belts (406, 408) before combining with the discrete chassis (102) at the nip (316) between the roll (318) and the carrier apparatus (308).

Referring to Figure 5A, once the chassis (102) is transferred for combining with the continuous front and back belts (406, 408), the continuous first substrate layer (462) having greater width than the continuous second substrate layer (464) may be folded, the folded portion of the continuous first substrate layer defining a continuous fold over (466FO) region. The continuous fold over (466FO) region may sandwich the continuous second substrate layer (464) with the continuous first substrate layer as in Figure 5A. The cross machine dimension of the continuous fold over (466FO) region may substantially match that of the excessive dimension of the continuous first substrate layer (462) compared to the continuous second substrate layer (464), to eventually provide belt structures as in Figures 3A and 3B. Alternatively, the cross machine dimension of the continuous fold over (466FO) region may be smaller than that of the excessive dimension of the continuous first substrate layer (462) compared to the continuous second substrate layer (464), as in Figure 5A. Concurrently with adhering the continuous fold over (466FO) region to the continuous second substrate layer (464), the front and back longitudinal edge regions of the discrete chassis (102) may be intermittently sandwiched between the continuous fold over (466FO) region and the continuous second substrate layer (464). This may secure the attachment of the chassis (102) to the continuous front and back belts (406, 408), and further cover the front and back longitudinal edges of the chassis (102).

With reference to Figures 4 and 5B, a continuous length of absorbent articles (400) are defined by multiple discrete chassis (102) spaced from each other along the machine direction and connected with each other by the continuous back belt (408) and the continuous front belt (406). Referring to Figures 4, 5A and 5B, the continuous length of articles (400) advances from the nip (316) to a folding apparatus (332). At the folding apparatus (332), each chassis (102) is folded in the cross machine direction along a lateral axis (126) to place the front waist region (116), and specifically, the inner, body facing surface (312) into a facing, surface to surface orientation with the inner, body facing surface (312) of the back waist region (118). The folding of the chassis (102) also positions the body facing surface (312) of the continuous back belt (408) extending between each chassis (102) in a facing relationship with the body facing surface (312) of the continuous front belt (406) extending between each chassis (102). Accordingly, this overlays the continuous front belt (406) and the continuous back belt (408). Either edges of the front belt (106) and the back belt (108) may be matched. When the chassis (102) is folded at the cross machine middle point of the assembly, then the distal edges of the continuous front and back belts are matched.

As shown in Figures 4, 5B, and 5C, the folded discrete chassis (102) connected with the continuous front and back belts (406, 408) are advanced from the folding apparatus (332) to a bonder module (334). The bonder module (334) operates to bond an overlap area (362), thus creating discrete bonds (336a, 336b). The overlap area (362) includes a portion of the continuous back belt (408) extending between each chassis (102) and a portion of the continuous front belt (406) extending between each chassis (102). As shown in Figures 4 and 5D, a continuous length of articles are advanced from the bonder module (334) to a knife roll (338) where the continuous front belt (406) and the continuous back belt (408) are cut along the cross machine direction between adjacent bonds (336a, 336b) to create discrete finished pant articles (100). As such, one bond (336a) may correspond with and form a first side seam (178) on an article (100), and the other bond (336b) may correspond with and form a second side seam (180) on a subsequently advancing article.

With reference to Figure 4, the converting apparatus may include a bonder module (334) to create bonds (336a, 336b). The bonder module (334) is configured to intermittently seam the obtained assembly by directing a jet of heated air to at least partially melt the substrates of the continuous front belt (406) and the substrates of the continuous back belt (408), and compressing the melted portion between an outer circumferential surface (370) of an anvil roll (368) and a pressing member (380).

As explained above and referring to Figures 3A, 3B, and Figure 5C, the regions of the assembly to be bonded may include regions made with six layers of substrates and four layers of substrates. The region made with six layers include the continuous fold over (466FO) regions coming from both the continuous front and back belts (406, 408). In view of such difference in the number of layers of substrates of the assembly, the seam strength may vary when the six layer region and four layer region are seamed in just one seaming step utilizing compression surfaces that are homogenous along the longitudinal dimension of the seam. When the seam strength of the resulting article is varied along the longitudinal dimension of the side seam, this may result in a greater Top Bottom Difference, a too high Belt Maximum Peel Strength, or a too low Belt Minimum Peel Strength. The process for forming hot air bonds of the present invention may include various methods for preventing such difference in seam strength, while also keeping the seam strength within a scope which enables the side seams to tolerate normal usage conditions, and also being easy to open after use. Specifically, the present process may provide relatively lower bonding pressure against the six layer region, compared to the four layer region. Alternatively or additionally, the present process may provide bonding pressure for more than once to the four layer region, thus compensating for the otherwise lower bonding pressure applied to the four layer region.

The obtained assembly at the stage of Figure 5C may be seamed by directing a jet of heated air to at least partially melt the substrates of the continuous front belt (406) and the substrates of the continuous back belt (408), and then compressing the melted portion between an outer circumferential surface (370) of an anvil roll (368) and a pressing member (380) wherein the compression is conducted for at least 2 times. The compressing may comprise a first compression and a second compression, wherein the region of compressing of the first compression and second compression vary over the longitudinal dimension of the side seam.

For example, Figure 6A1 shows a schematic side view of a first embodiment of a bonder module (334) that may be utilized with the methods and apparatuses herein. As shown in Figure 6A1, the bonder module (334) may include a seaming drum (364R) and a compressing stage (335) located adjacent the seaming drum (364R). Referring to Figure 6B1, the seaming drum (364R) may include an outer circumferential surface (376) and adapted to rotate about an axis of rotation (374). The seaming drum (364R) may also include a plurality of seaming stations (348) positioned radially inward from the outer circumferential surface (376) and the drum apertures (366), as in Figure 6B3. Each seaming station (348) may include a hot air nozzle (378) which directs a jet of the heated air through the drum aperture (366) to at least partially melt the overlap area (362) of the substrates of the continuous front belt (406) and the substrates of the continuous back belt (408). Although the seaming drum (364R) shown in Figure 6B1 includes six seaming stations (348), the seaming drum (364) may be configured to include more or less than six seaming stations (348). The assembly may be rotated about the seaming drum (364R) in order to provide sufficient time for the melting, such that the melted portion may be compressed to provide the side seam of the resulting article to have continuity of melted material along the substantial entirety of its longitudinal dimension. Referring to Figures 6A1 and 6B1, the hot air nozzles (378) may be actively directing heated air only in the active regions (390) of the seaming drum (364R). Referring to Figure 6A1, the compressing stage (335) may be located shortly after leaving the seaming drum (364R). The compressing stage (335) may comprise one set of an anvil roll (368) and a pressing member (380) that engage with each other. The anvil roll (368) includes an outer circumferential surface (370) and is adapted to rotate about an axis of rotation (372). The pressing member (380) may comprise a pair or projections (422) for engaging with the outer circumferential surface (370) for forming a pair of side seams adjacent each other. By providing a compression stage (335) independent of the seaming drum (364R), the anvil roll (368) and the pressing member (380) may each be adjusted according to the type of assembly to seam. This is advantageous in that assemblies made of various types of substrates and various sizes may be seamed without the need to fabricate the seaming drum (364R). The outer circumferential surface (370) of the anvil roll (368) and the projection (422) of the pressing member (380) to engage with the outer circumferential surface (370) may have configurations for providing varying bonding pressure, which is discussed in detail below.

In other examples, Figures 6A2 and 6A3 show schematic side views of a second embodiment and a third embodiment, respectively, of a bonder module (334). Similar to the first embodiment, the second and third embodiments shown in Figures 6A2 and 6A3 may include the seaming drum (364R) as in Figure 6B1, while having a compression stage (335) of different configuration. Referring to Figure 6A2, the compressing stage (335) may be located shortly after leaving the seaming drum (364R), and the compressing stage (335) may comprise two sets of engaging anvil rolls (368) and pressing members (380). Referring to Figure 6A3, the compressing stage (335) may be located shortly after leaving the seaming drum (364R), and the compressing stage (335) may comprise one anvil roll (368) which engages with two pressing members (380). By providing a compression stage (335) independent of the seaming drum (364R) and further with more than one compression site, the multiple compression sites in Figures 6A2 and 6A3 may be configured to press against different portions of the assembly that may have different caliper or layers of nonwovens, and/or press against some portions more than once. This is also advantageous in that assemblies made of various types of substrates and various sizes may be seamed by changing one or more of the anvil rolls (368) and pressing members (380) without the need to fabricate the seaming drum (364R). The outer circumferential surface (370) of the anvil roll (368) and the projection (422) of the pressing member (380) to engage with the outer circumferential surface (370) may have configurations for providing varying bonding pressure, which is discussed in detail below.

Figure 6A4 shows a schematic side view of a fourth embodiment of a bonder module (334) that may be utilized with methods and apparatuses which are different from those discussed above. As shown in Figure 6A4, the bonder module (334) may include a seaming drum (364C) and an anvil roll (368) configured to engage with the pressing member (380) included in the seaming drum (364C), wherein the engagement is configured to happen immediately before leaving the seaming drum (364C), and an additional compressing stage (335). Referring to Figure 6B2 and 6B3, the seaming drum (364C) may include an outer circumferential surface (376) and adapted to rotate about an axis of rotation (374) wherein the seaming drum (364C) is equipped with a cam function to enable the seaming station (348) to move radially inward and outward. For example, referring to Figure 6A4 and 6B2, the seaming stations (348) may receive the continuous front and back belts (406, 408) when positioned radially inward from the outer circumferential surface (376), and after providing sufficient time for melting in the active regions (390), then move radially outward such that the pressing member (380) may engage with the anvil roll (368) immediately before leaving the seaming drum (364C). The once seamed continuous front and back belts (406, 408) may be sent to the compressing stage (335). The compressing stage (335) may comprise one set of an anvil roll (368) and a pressing member (380) which engage with each other. Figure 6A5 shows a schematic side view of a fifth embodiment of a bonder module (334). Similar to the fourth embodiment, the fifth embodiment shown in Figure 6A5 may include the seaming drum (364C) as in Figure 6B2, while having compression stages (335) of different configuration. There may be a compressing stage (335) comprising two sets of engaging anvil rolls (368) and pressing members (380). By providing one pressing site within the seaming drum (364C) and another pressing site(s) in the compression stage (335), the multiple compression sites in Figures 6A4 and 6A5 may be configured to press against different portions of the substrate that may have different caliper or layers of nonwovens, and/or press against some portions more than once. This is also advantageous in that the assemblies made of various types of substrates and various sizes may be seamed default once within the seaming drum (364C), and further changing one or more of the anvil roll (368) and pressing members (380) to meet further needs of the assembly. The outer circumferential surface (370) of the anvil roll (368) and the projection (422) of the pressing member (380) to engage with the outer circumferential surface (370) may have configurations for providing varying bonding pressure, which is discussed in detail below.

Referring to Figures 6A1-6A5, the outer circumferential surface (370) of the anvil roll (368) engaging with the seaming drum (364C) as well as those in the compressing stage (335) may take various configurations for effectively seaming regions having more and less material. Referring to Figure 6C, the outer circumferential surface of the anvil roll may comprise a grooved region (370G) having a repetition of intermittently indented surfaces alternating in the cross machine direction. The outer circumferential surface may be made completely with the groove region (370G) (not shown). Alternatively, the outer circumferential surface may be made with a groove region (370G) and a flat region (370F) devoid of the intermittent indented surfaces, as in Figure 6C. The indentations in the grooved region (370G) provide vacancy of applied pressure when compressed against the pressing member (380), thus the seaming strength may be adjusted. The seam made by the grooved region (370G) may have decreased strength compared to the seam made by the flat region (370F). In the ring-like elastic belt (104) of the present invention, the regions having more material may be pressed against each other with higher pressing due to the thickness of the compressed region, and thus result in unnecessarily high seam strength. On the other hand, those regions having less material may result in a low seam strength. In other words, if the seam were compressed with the same pressure along its longitudinal dimension, this may result in a gradation of seam strength along the seam. As such, the fold over (162FO) region may be configured to meet the grooved region (370G) of the outer circumferential surface (370) of the anvil roll (368) to provide lower compression pressure.

Referring to Figure 6D, the compressing stage (335) also includes a pressing member (380) to compress the partially melted overlap area against the outer circumferential surface (370) of the anvil roll (368). The pressing member (380) may be substantially rectangular in shape and comprising two projections (422) for engaging with the machine direction center of the melted portions to form a first bond (336a) and a second bond (336b) (not shown). The projections may be flat. Referring to Figure 6D, each of the projections (422) may comprise a regular surface (423) and an indented surface (421), wherein the regular surface (423) is positioned to engage with the outer circumferential surface of the anvil roll with a smaller distance (H) thus providing higher compression pressure, while the intended surface (421) provides relatively lower compression pressure. The fold over (162FO) region may be configured to meet the indented surface (421) of the projection (422). The height difference (H) between the indented surface (421) and the regular surface (423) may be from about 0.1mm to about 0.6mm, or from about 0.2mm to about 0.4mm. The surfaces may be slightly tapered towards the machine direction to avoid being easily worn.

As previously mentioned above with reference to Figures 4, 5C and 5D, once the bonds (336a, 336b) are formed, the articles (400) advance in the machine direction to a knife roll (338) where the continuous front and back belts (406, 408) are cut along the cross machine direction between the bonds (336a, 336b) to create a first side seam (178) on an article (100) and a second side seam (180) on a subsequently advancing article. The pressing member (380) and anvil roll surface (370) may be coated with, for example, a plasma coating, polytetrafluoroethylene, or silicone.

Measurements

1. Preparation of Finished Product Specimen

Specimen for the measurements hereinbelow are obtained from a finished wearable article sample, or ring-like elastic belt (104) sample, unless otherwise specified. To obtain a belt specimen from a finished wearable article sample, the belt is detached from the chassis (102) by hand.

Specimen are obtained from 6 (six) finished wearable articles from the same area of each article for each set of measurement. Specimen are pre-conditioned in a room maintained at 23 $\pm$ 2 °C and 50 $\pm$ 5 % relative humidity, for at least 2 hours prior to testing. All testing is performed in a room maintained at 23 $\pm$ 2 °C and 50 $\pm$ 5 % relative humidity.

2. Belt Minimum Peel Strength, Belt Maximum Peel Strength, Top Bottom Difference

As equipment, MTS Criterion C42 running TestWorks 4 Software with standard tensiometer jaw or equivalent is used.

4 (four) unique seam specimen are obtained from one belt specimen by cutting off the top (distal) edges and bottom (proximal) edges of the opposed longitudinal edges of the left and right seams in a longitudinal dimension (cross machine direction) of 25mm and a lateral dimension (machine direction) of 50mm by scissors. Care is taken such that, when an edge of the seam is discontinuous, such discontinuous portion is avoided and the continuous portion of the seam is sampled. Each of the 4 unique seam specimen from one belt specimen are provided identifiable as "top left", "top right", "bottom left", and "bottom right".

(1) The seam specimen is set such that the lateral direction of the belt matches the vertical direction of the equipment. The seam specimen is clamped between the upper and lower tensiometer jaws as straight as possible without applying pretension.

(2) The elongation measurement is taken from the point where the force curve leaves the zero line.

(3) A constant rate of extension of 460mm/min is applied.

(4) Pull the seam specimen until the seam is completely separated. Record the peak force (N/25mm).

(5) For each of the "top left", "top right", "bottom left", and "bottom right" specimen, the average peak force of the 6 values of the 6 seam specimen are obtained and averaged, respectively, wherein each averaged value is named FTL, FTR, FBL, and FBR. The smallest of FTL, FTR, FBL, and FBR is the Belt Minimum Peel Strength, and the greatest of FTL, FTR, FBL, and FBR is the Belt Maximum Peel Strength.

(6) The Top Bottom Difference is obtained as such:

$$\left| \{(FTL + FTR) - (FBL + FBR)\} \div (FBL + FBR) \right| \quad (\%)$$

3. Average Peel Strength

As equipment, MTS Criterion C42 running TestWorks 4 Software with standard tensiometer jaw or equivalent is used.

The left and right seam specimen are obtained from one belt specimen by cutting off the left and right seams for the entire longitudinal length of the seam and at a lateral dimension (machine direction) of 50mm by scissors. The left and right seams are subject to following measurement separately.

(1) The specimen is set such that the lateral direction of the belt matches the vertical direction of the equipment. The distal edge of the specimen is clamped between the upper and lower tensiometer jaws as straight as possible without applying pretension.
(2) A constant rate of extension of 2000 mm/min is applied.
(3) Pull the specimen until the seam is completely separated. The force distribution up to 0.01N preciseness is recorded.
(4) The force (N) of highest frequency, as in Figure 7, is recorded. The highest frequency from the left seam specimen and right seam specimen is averaged to obtain the Average Peel Strength (N).

4. Material Breaking Point

As equipment, MTS Criterion C42 running TestWorks 4 Software with standard tensiometer jaw or equivalent is used. For this measurement, raw material of the first substrate layer (162) and the raw material of the second substrate layer (164) is used. According to how the substrate is planned to be assembled, the substrate is cut in a lateral dimension (machine direction) of 25mm and longitudinal dimension (cross machine direction) of 50mm to provide a specimen. Thirty (30) specimens are obtained using different lots of substrate layers, or different regions of substrate layers.

(1) The specimen is set such that the longitudinal direction in which the layers are planned to be introduced to the belt matches the vertical direction of the equipment. The layers are clamped to leave 25mm initial gauge length. The specimen is clamped between the upper and lower tensiometer jaws as straight as possible without applying pretension.
(2) A constant rate of extension of 2000mm/min is applied.
(3) Pull the specimen until the specimen is completely broken. The force distribution up to 0.01N preciseness is recorded.
(4) The force (N) of highest frequency, as in Figure 7, is the Material Breaking Point (N).

EXAMPLE

Examples 1-5 as identified in Table 1 below, were subject to various measurements and in-use tests. Examples 1-3 were marketed products manufactured not according to manufacturing methods of the present invention. Examples 4-5 were manufactured according to manufacturing methods of the present invention. All Examples were made by very similar raw materials and elastic profiles, which were within the manufacturer's specifications.

The Examples were subject to the measurements detailed above for obtaining the Belt Minimum Peel Strength, Belt Maximum Peel Strength, Top Bottom Difference, and recorded in Table 1 below. Further, the Examples were subject to an in-use test detailed below.

In-use Test

30 panelists (10 panelists each in the United States, Japan, and the United Kingdom) who were caregivers of babies of 6-48 months in age and 9-14kg in weight, and being a pant product user, with mixture of boy/girl babies, were provided enough test products. Each panelist used test products and tore the side seam according to their usual habit. Most of the time, the baby wearer was standing while tear opening the side seam.

The panelists were requested to observe if there was any horizontal tearing during the opening. By horizontal tearing, what is meant is the phenomena of the tearing line sidetracking in a direction away from the side seam, and rather ripping the elastic belt. Horizontal tearing includes various directions of tearing, not necessarily horizontal, so long as the side seam tearing is sidetracked or derailed. The incident of horizonal tearing is recorded by the panelist.

Table 1

| Example | Product name / Country / Lot# | Horizontal tearing (%) | FTL (N/25mm) | FTR (N/25mm) | FBL (N/25mm) | FBR (N/25mm) | Top Bottom Difference (%) |
|---|---|---|---|---|---|---|---|
| 1 | Pampers Premium Care Pants / Japan / 80192022PO | 35 | 15.0 | 13.6 | 12.5 | 11.7 | 18.2% |
| 2 | Pampers Premium Care Pants / Japan / 72462022QO | 61 | 19.5 | 17.3 | 16.8 | 17.7 | 6.7% |
| 3 | Pampers Premium Protection Active Fit Pants / Poland / 81054518J3 | 65 | 19.3 | 12.6 | 16.5 | 15.8 | 1.2% |
| 4 | Prototype / China / 8177A869V0-Dayuan | 21 | 13.2 | 13.1 | 14.2 | 14.4 | 8.0% |
| 5 | Prototype / China / 8177A869V0-Wisdom | 20 | 14.7 | 15.3 | 16.1 | 17.8 | 11.5% |

Examples 1-3 within product specifications at the time of manufacture provided side seams of various peel strength depending on the manufacturing site and/or lot, namely, stable side seam quality could not be obtained Due to such unstable quality, there were significant differences in incident of horizontal tearing, leading to low consumer acceptance.

Examples 4-5 manufactured according to the method of the present invention provided more stable side seam quality. Examples 4-5 according to the present invention had the same product specifications as Examples 1-3 except Belt Minimum Peel Strength, Belt Maximum Peel Strength, and Top Bottom Difference. Further, there were significantly less incidents of horizontal tearing in the in-use test for Examples 4-5. The parameters of the present invention provide a good predictability of consumer acceptance.

## Claims

1.    A ring-like elastic belt (104) having a longitudinal direction and a lateral direction comprising:

a front belt (106) and a back belt (108), each of the front belt (106) and back belt (108) being an elastic laminate comprising a first substrate layer (162) , a second substrate layer (164) , and a plurality of elastic bodies (168) joined between the first substrate layer (162) and the second substrate layer (164) , each of the front belt (106) and the back belt (108) further comprising a fold over (162FO) of at least one of the first substrate layer (162) and the second substrate layer (164) wherein the fold over (162FO) of the front belt (106) has a longitudinal dimension shorter than the front belt (106), and the fold over (162FO) of the back belt (108) has a longitudinal dimension shorter than the front belt (106), a pair of side seams which join the lateral edges of the front belt (106) and the back belt (108) such that the front fold over (162FO) and the back fold over (162FO) cooperatively define a distal edge of the ring-like elastic belt (104), the side seam made by a hot air bond which at least partially melts material of the elastic laminate, the side seam having continuity of the melted material along the substantial entirety of its longitudinal dimension;
wherein the side seam has a Belt Minimum Peel Strength of at least about 6N/25mm, a Belt Maximum Peel Strength of no more than 18N/25mm, and a Top Bottom Difference of no more than 15 %, according to the measurements herein.

2.    The belt of Claim 1 wherein the side seam has an Average Peel Strength and the combined first and second substrate layer (164) has a Material Breaking Point according to measurements herein, wherein the Average Peel Strength is from about 20 % to about 50 % of the Material Breaking Point.

3. The belt of any of the preceding claims wherein each of the first substrate layer (162) and the second substrate layer (164) has a Material Breaking Point according to the measurements herein, wherein the Material Breaking Point is less than about 8N.

4. The belt of any of the preceding claims wherein the elastic bodies (168) are disposed in a manner such that at least one elastic body from the front belt (106) and at least one elastic body from the back belt (108) overlap with each other at the side seam.

5. The belt of any of the preceding claims wherein the elastic bodies (168) are intermittently disposed in varying intervals, wherein the pattern of intervals of elastic bodies (168) disposed on the front belt (106) is different from the pattern of intervals of the elastic bodies (168) disposed on the back belt (108).

6. The belt of any of the preceding claims wherein the belt has a maximum basis weight of at least about 90gsm.

7. The belt of any of the preceding claims wherein the longitudinal dimension of the front belt (106) is different from the longitudinal dimension of the back belt (108), wherein the distal edges of the front belt (106) and back belt (108) are matched with each other.

8. The belt of any of the preceding claims wherein the first substrate layer (162) and the second substrate layer (164) are nonwoven materials having difference in at least one of basis weight, stiffness, and texture.

9. The belt of Claim 8 wherein at least one of the first substrate layer (162) and the second substrate layer (164) has a plurality of openings or bondings.

10. The belt of Claim 8 wherein the first substrate layer (162) is a nonwoven fabric made of air-through carded fibers.

11. A wearable article comprising a center chassis (102) and a ring-like elastic belt (104) according to any of the preceding claims wherein the center chassis (102) is bridged from the center of the front belt (106) to the center of the back belt (108).

12. A method of manufacturing the ring-like elastic belt (104) of any of claims 1 to 10, the method comprising the steps of:

   advancing a continuous first substrate layer (462) in a machine direction, the continuous first substrate layer (462) having a first surface and an opposing second surface and defining a width in a cross machine direction;
   advancing a continuous second substrate layer (464) in the machine direction, the continuous second substrate layer (464) having a first surface and an opposing second surface and having a smaller width than the continuous first substrate layer (462);
   advancing a plurality of elastic bodies (168) in the machine direction in a stretched state; joining the elastic bodies (168) between the first surface of the continuous first substrate layer (462) and the first surface of the continuous second substrate layer (464);
   cutting the assembly in the machine direction where the continuous first substrate layer (462) and continuous second substrate layer (464) are overlayed to define a continuous front belt (406) and a continuous back belt (408);
   folding the continuous first substrate layer (462) along the machine direction of at least one of the continuous front belt (406) and the continuous back belt, the folded portion of the continuous first substrate layer (462) defining a continuous fold over (466FO) region; overlaying the continuous front belt (406) and the continuous back belt (408) such that the cross machine edges of the continuous front belt (406) and the continuous back belt (408) are matched; and
   intermittently seaming the obtained assembly by directing a jet of heated air to at least partially melt the substrates of the continuous front belt (406) and the substrates of the continuous back belt (408), and compressing the melted portion between an outer circumferential surface (370) of an anvil roll (368) and a pressing member (380); wherein

      i) the outer circumferential surface of the anvil roll comprises a grooved region (370G) having a repetition of intermittently indented surfaces alternating in the cross machine direction and a flat region (370F) devoid of the intermittent indented surfaces, wherein the continuous fold over (466FO) region is configured to meet the grooved region (370G) of the anvil roll upon compression; or

ii) the pressing member (380) comprises a regular surface (423) and an indented surface (421) , wherein the continuous fold over (466FO) region is configured to meet the indented surface of the pressing member (380); or

iii) the compression is conducted for at least 2 times.

13. The method of Claim 12, option i) wherein the pressing member (380) comprises a regular surface (423) and an indented surface (421), wherein the continuous fold over (466FO) region is configured to meet the indented surface of the pressing member (380)

14. The method of Claim 12, option iii) wherein the compression comprises a first compression and a second compression, wherein the region of compressing of the first compression and second compression vary over the longitudinal dimension of the side seam.

**Patentansprüche**

1. Ringartiger Gummibandbund (104), der eine Längsrichtung und eine Querrichtung aufweist, umfassend:

   einen vorderseitigen Bund (106) und einen rückseitigen Bund (108), wobei jeder des vorderseitigen Bundes (106) und des rückseitigen Bundes (108) ein Gummibandlaminat ist, umfassend eine erste Substratschicht (162), eine zweite Substratschicht (164) und eine Vielzahl von Gummibandkörpern (168), die zwischen der ersten Substratschicht (162) und der zweiten Substratschicht (164) verbunden sind, jedes des vorderseitigen Bundes (106) und des rückseitigen Bundes (108) ferner umfassend eine Faltung (162FO) von wenigstens einer der ersten Substratschicht (162) und der zweiten Substratschicht (164), wobei die Faltung (162FO) des vorderseitigen Bundess (106) eine Längsabmessung aufweist, die kürzer als der vorderseitige Bund (106) ist, und die Faltung (162FO) des rückseitigen Bundes (108) eine Längsabmessung aufweist, die kürzer als der vorderseitige Bund (106) ist, ein Paar Seitennähte, die die Seitenränder des vorderseitigen Bundes (106) und des rückseitigen Bundes (108) verbinden, sodass die vorderseitige Faltung (162FO) und die rückseitige Faltung (162FO) zusammenwirkend einen distalen Rand des ringartigen Gummibandbundes (104) definieren, wobei die Seitennaht durch eine Heißluftbindung hergestellt wird, die wenigstens teilweise Material des Gummibandlaminats schmilzt, wobei die Seitennaht eine Kontinuität des geschmolzenen Materials entlang der wesentlichen Gesamtheit ihrer Längsabmessung aufweist;
   wobei die Seitennaht eine minimale Bundschälfestigkeit von wenigstens etwa 6 N/25 mm, eine maximale Bundschälfestigkeit von nicht mehr als 18 N/25 mm und eine Differenz von oben nach unten von nicht mehr als 15 % nach den Messungen hierin aufweist.

2. Bund nach Anspruch 1, wobei die Seitennaht eine durchschnittliche Schälfestigkeit aufweist und die kombinierte erste und zweite Substratschicht (164) eine Materialsollbruchstelle gemäß den Messungen hierin aufweist, wobei die durchschnittliche Schälfestigkeit von etwa 20 % bis etwa 50 % der Materialsollbruchstelle beträgt.

3. Bund nach einem der vorstehenden Ansprüche, wobei jede der ersten Substratschicht (162) und der zweiten Substratschicht (164) eine Materialsollbruchstelle nach den Messungen hierin aufweist, wobei die Materialsollbruchstelle weniger als etwa 8 N beträgt.

4. Bund nach einem der vorstehenden Ansprüche, wobei die Gummibandkörper (168) so eingerichtet sind, dass sich wenigstens ein Gummibandkörper von dem vorderseitigen Bund (106) und wenigstens ein Gummibandkörper von dem rückseitigen Bund (108) an der Seitennaht überlappen.

5. Bund nach einem der vorstehenden Ansprüche, wobei die Gummibandkörper (168) unterbrochen in variierenden Abständen eingerichtet sind, wobei sich das Muster von Abständen von Gummibandkörpern (168), die an dem vorderseitigen Bund (106) eingerichtet sind, von dem Muster von Abständen der Gummibandkörper (168), die an dem rückseitigen Bund (108) eingerichtet sind, unterscheidet.

6. Bund nach einem der vorstehenden Ansprüche, wobei der Bund ein maximales Flächengewicht von wenigstens etwa 90 g/m$^2$ aufweist.

7. Bund nach einem der vorstehenden Ansprüche, wobei sich die Längsabmessung des vorderseitigen Bundes (106) von der Längsabmessung des rückseitigen Bundes (108) unterscheidet, wobei die distalen Ränder des vorderseitigen Bundes (106) und des rückseitigen Bundes (108) übereinstimmen.

8. Bund nach einem der vorstehenden Ansprüche, wobei die erste Substratschicht (162) und die zweite Substratschicht (164) Vliesmaterialien sind, die eine Differenz in wenigstens einem von Flächengewicht, Steifigkeit und Textur aufweisen.

9. Bund nach Anspruch 8, wobei wenigstens eine der ersten Substratschicht (162) und der zweiten Substratschicht (164) eine Vielzahl von Öffnungen oder Bindungen aufweist.

10. Bund nach Anspruch 8, wobei die erste Substratschicht (162) ein Vliesstoff ist, der aus luftkardierten Fasern hergestellt ist.

11. Tragbarer Artikel, umfassend eine mittlere Grundeinheit (102) und einen ringartigen Gummibandbund (104) nach einem der vorstehenden Ansprüche, wobei die mittlere Grundeinheit (102) von der Mitte des vorderseitigen Bundes (106) zu der Mitte des rückseitigen Bundes (108) überbrückt ist.

12. Verfahren zum Fertigen des ringartigen Gummibandbundes (104) nach einem der Ansprüche 1 bis 10, das Verfahren umfassend die Schritte:

    Vorwärtsbewegen einer ununterbrochenen ersten Substratschicht (462) in einer Maschinenlaufrichtung, wobei die ununterbrochene erste Substratschicht (462) eine erste Oberfläche und eine entgegengesetzte zweite Oberfläche aufweist und eine Breite in einer Maschinenquerrichtung definiert;
    Vorwärtsbewegen einer ununterbrochenen zweiten Substratschicht (464) in der Maschinenlaufrichtung, wobei die ununterbrochene zweite Substratschicht (464) eine erste Oberfläche und eine entgegengesetzte zweite Oberfläche aufweist und eine kleinere Breite als die ununterbrochene erste Substratschicht (462) aufweist;
    Vorwärtsbewegen einer Vielzahl von Gummibandkörpern (168) in der Maschinenlaufrichtung in einem gedehnten Zustand;
    Verbinden der Gummibandkörper (168) zwischen der ersten Oberfläche der ununterbrochenen ersten Substratschicht (462) und der ersten Oberfläche der ununterbrochenen zweiten Substratschicht (464);
    Spanen der Anordnung in der Maschinenlaufrichtung, wo die ununterbrochene erste Substratschicht (462) und die ununterbrochene zweite Substratschicht (464) überlagert sind, um einen ununterbrochenen vorderseitigen Bund (406) und einen ununterbrochenen rückseitigen Bund (408) zu definieren;
    Falten der ununterbrochenen ersten Substratschicht (462) entlang der Maschinenlaufrichtung von wenigstens einem des ununterbrochenen vorderseitigen Bundes (406) und des ununterbrochenen rückseitigen Bundes, wobei der gefaltete Abschnitt der ununterbrochenen ersten Substratschicht (462) einen ununterbrochenen Faltungsbereich (466FO) definiert;
    Überlagern des ununterbrochenen vorderseitigen Bundes (406) und des ununterbrochenen rückseitigen Bundes (408), sodass die Quermaschinenränder des ununterbrochenen vorderseitigen Bundes (406) und des ununterbrochenen rückseitigen Bundes (408) übereinstimmen; und
    unterbrochenes Vernähen der erhaltenen Anordnung durch Richten eines Strahls von Warmluft, um die Substrate des ununterbrochenen vorderseitigen Bundes (406) und die Substrate des ununterbrochenen rückseitigen Bundes (408) wenigstens teilweise zu schmelzen und den geschmolzenen Abschnitt zwischen einer äußeren Umfangsoberfläche (370) einer Ambosswalze (368) und einem Druckelement (380) zu komprimieren; wobei

        i) die äußere Umfangsoberfläche der Ambosswalze einen gerillten Bereich (370G), der eine Wiederholung von unterbrochen eingebuchteten Oberflächen aufweist, die sich in der Maschinenquerrichtung abwechseln, und einen flachen Bereich (370F), der frei von den unterbrochen eingebuchteten Oberflächen ist, umfasst, wobei der ununterbrochene Faltungsbereich (466FO) konfiguriert ist, um dem gerillten Bereich (370G) der Ambosswalze bei einer Kompression zu begegnen; oder
        ii) das Druckelement (380) eine regelmäßige Oberfläche (423) und eine eingebuchtete Oberfläche (421) umfasst, wobei der ununterbrochene Faltungsbereich (466FO) konfiguriert ist, um der eingebuchteten Oberfläche des Druckelements (380) zu begegnen; oder
        iii) die Kompression wenigstens 2 Mal durchgeführt wird.

13. Verfahren nach Anspruch 12, Option i) wobei das Druckelement (380) eine regelmäßige Oberfläche (423) und eine eingebuchtete Oberfläche (421) umfasst, wobei der ununterbrochene Faltungsbereich (466FO) konfiguriert ist, um der eingebuchteten Oberfläche des Druckelements (380) zu begegnen.

**14.** Verfahren nach Anspruch 12, Option iii) wobei die Kompression eine erste Kompression und eine zweite Kompression umfasst, wobei der Kompressionsbereich der ersten Kompression und der zweiten Kompression über die Längsabmessung der Seitennaht variiert.

## Revendications

**1.** Ceinture élastique annulaire (104) ayant une direction longitudinale et une direction latérale comprenant :

une ceinture avant (106) et une ceinture arrière (108), chacune de la ceinture avant (106) et de la ceinture arrière (108) étant un stratifié élastique comprenant une première couche de substrat (162), une seconde couche de substrat (164), et une pluralité de corps élastiques (168) reliés entre la première couche de substrat (162) et la seconde couche de substrat (164), chacune de la ceinture avant (106) et de la ceinture arrière (108) comprenant en outre un rabat (162FO) d'au moins l'une de la première couche de substrat (162) et de la seconde couche de substrat (164) dans laquelle le rabat (162FO) de la ceinture avant (106) a une dimension longitudinale plus courte que la ceinture avant (106), et le rabat (162FO) de la ceinture arrière (108) a une dimension longitudinale plus courte que la ceinture avant (106), une paire de coutures latérales qui relient les bords latéraux de la ceinture avant (106) et de la ceinture arrière (108) de telle sorte que le rabat avant (162FO) et le rabat arrière (162FO) définissent en coopération un bord distal de la ceinture élastique annulaire (104), la couture latérale étant réalisée par une liaison à l'air chaud qui fait fondre au moins partiellement le matériau du stratifié élastique, la couture latérale présentant une continuité du matériau fondu le long de la quasi-totalité de sa dimension longitudinale ;
dans laquelle la couture latérale a une résistance au pelage minimale de ceinture d'au moins environ 6N/25 mm, une résistance au pelage maximale de ceinture n'excédant pas 18N/25 mm, et une différence haut-bas ne dépassant pas 15 %, selon les mesures dans le présent document.

**2.** Ceinture selon la revendication 1, dans laquelle la couture latérale a une résistance au pelage moyenne et la première et la seconde couche de substrat (164) combinées ont un point de rupture de matériau selon les mesures dans le présent document, dans laquelle la résistance au pelage moyenne va d'environ 20 % à environ 50 % du point de rupture de matériau.

**3.** Ceinture selon l'une quelconque des revendications précédentes, dans laquelle chacune de la première couche de substrat (162) et de la seconde couche de substrat (164) a un point de rupture de matériau selon les mesures dans le présent document, dans laquelle le point de rupture de matériau est inférieur à environ 8N.

**4.** Ceinture selon l'une quelconque des revendications précédentes, dans laquelle les corps élastiques (168) sont disposés d'une manière telle qu'au moins un corps élastique de la ceinture avant (106) et au moins un corps élastique de la ceinture arrière (108) se chevauchent l'un l'autre au niveau de la couture latérale.

**5.** Ceinture selon l'une quelconque des revendications précédentes, dans laquelle les corps élastiques (168) sont disposés de manière intermittente dans des intervalles variables, dans laquelle le motif d'intervalles de corps élastiques (168) disposés sur la ceinture avant (106) est différent du motif d'intervalles des corps élastiques (168) disposés sur la ceinture arrière (108).

**6.** Ceinture selon l'une quelconque des revendications précédentes, dans laquelle la ceinture a une masse surfacique maximale d'au moins environ 90 g/m$^2$.

**7.** Ceinture selon l'une quelconque des revendications précédentes, dans laquelle la dimension longitudinale de la ceinture avant (106) est différente de la dimension longitudinale de la ceinture arrière (108), dans laquelle les bords distaux de la ceinture avant (106) et de la ceinture arrière (108) sont appariés l'un à l'autre.

**8.** Ceinture selon l'une quelconque des revendications précédentes, dans laquelle la première couche de substrat (162) et la seconde couche de substrat (164) sont des matériaux non tissés présentant une différence au moins de masse surfacique, de rigidité ou de texture.

**9.** Ceinture selon la revendication 8, dans laquelle au moins l'une de la première couche de substrat (162) et de la seconde couche de substrat (164) a une pluralité d'ouvertures ou de liaisons.

**10.** Ceinture selon la revendication 8, dans laquelle la première couche de substrat (162) est un tissu non tissé constitué de fibres cardées à passage d'air.

**11.** Article portable comprenant un châssis central (102) et une ceinture élastique annulaire (104) selon l'une quelconque des revendications précédentes, dans lequel le châssis central (102) est ponté du centre de la ceinture avant (106) au centre de la ceinture arrière (108).

**12.** Procédé de fabrication de la ceinture élastique annulaire (104) selon l'une quelconque des revendications 1 à 10, le procédé comprenant les étapes consistant à :

faire avancer une première couche de substrat continue (462) dans un sens machine, la première couche de substrat continue (462) présentant une première surface et une seconde surface opposée, et définissant une largeur dans un sens travers ;

faire avancer une seconde couche de substrat continue (464) dans le sens machine, la seconde couche de substrat continue (464) présentant une première surface et une seconde surface opposée et présentant une largeur plus petite que la première couche de substrat continue (462) ;

faire avancer une pluralité de corps élastiques (168) dans le sens machine dans un état étiré ;

relier les corps élastiques (168) entre la première surface de la première couche de substrat continue (462) et la première surface de la seconde couche de substrat continue (464) ;

découper l'ensemble dans le sens machine où la première couche de substrat continue (462) et la seconde couche de substrat continue (464) sont superposées pour définir une ceinture avant continue (406) et une ceinture arrière continue (408) ;

plier la première couche de substrat continue (462) le long du sens machine d'au moins l'une de la ceinture avant continue (406) et de la ceinture arrière continue, la partie pliée de la première couche de substrat continue (462) définissant une région de rabat continu (466FO) ;

superposer la ceinture avant continue (406) et la ceinture arrière continue (408) de telle sorte que les bords de sens travers de la ceinture avant continue (406) et de la ceinture arrière continue (408) sont appariés ; et

réaliser une couture intermittente de l'ensemble obtenu en dirigeant un jet d'air chauffé pour faire fondre au moins partiellement les substrats de la ceinture avant continue (406) et les substrats de la ceinture arrière continue (408), et en comprimant la partie fondue entre une surface circonférentielle externe (370) d'un rouleau enclume (368) et un élément de pressage (380) ;

dans lequel

i) la surface circonférentielle externe du rouleau enclume comprend une région rainurée (370G) présentant une répétition de surfaces dentelées par intermittence en alternance dans le sens travers et une région plate (370F) dépourvue de surfaces dentelées par intermittence, dans lequel la région de rabat continu (466FO) est conçue pour rejoindre la région rainurée (370G) du rouleau enclume lors de la compression ; ou

ii) l'élément de pressage (380) comprend une surface régulière (423) et une surface dentelée (421), dans lequel la région de rabat continu (466FO) est conçue pour rejoindre la surface dentelée de l'élément de pressage (380) ; ou

iii) la compression est effectuée au moins 2 fois.

**13.** Procédé selon la revendication 12, option i) dans lequel l'élément de pressage (380) comprend une surface régulière (423) et une surface dentelée (421), dans lequel la région de rabat continu (466FO) est conçue pour rejoindre la surface dentelée de l'élément de pressage (380).

**14.** Procédé selon la revendication 12, option iii) dans lequel la compression comprend une première compression et une seconde compression, dans lequel la région de compression de la première compression et de la seconde compression varie sur la dimension longitudinale de la couture latérale.

**FIG. 1**

FIG. 2

FIG. 3A

FIG. 3B

FIG. 4

EP 3 856 107 B1

462 170 170

464

102 312 312

464

170 312 466FO

170

170 102

462 406

312

466FO

408

## FIG. 5A

400

124 124 124

408 408 406 406

MD

126 102 102 102

CD 126

102 102 408 408 408

## FIG. 5B

400

362      362      362      362

406      406      406      406

CD

336a   336a   336a   336a 336b

336b    336b

102      102      102

MD

## FIG. 5C

336b   124   106   336a      336b   124   106   336a

MD

180    178      180    178

100   102      100   102

## FIG. 5D

334

400

MD

364R

FIG. 6A1

422

335

380

390

372    368

MD

334

364R

FIG. 6A2

335

422    380    422    380

390

372    368    372    368

MD

MD

FIG. 6A3

334

364R

MD

380

422

335

380

422

368

372

390

MD

FIG. 6A4

334

334

364C

MD

422

380

372

368

368

372

335

390

MD

FIG. 6A5

334

364C

MD

335

422   380   422   380

390

368   372   372   368   372   368

MD

FIG. 6B1

FIG. 6B2

FIG. 6B3

FIG. 6C

380

421

423

CD

422

H

421

423

422

MD

FIG. 6D

421

FREQUENCY

APS

MBP

FORCE (N)

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200617718 A
- US 2010063468 A
- WO 2011087503 A
- US 5628097 A
- WO 201673712 A
- WO 2012134988 A
- WO 2016029651 A
- WO 2016029652 A
- WO 2016029653 A
- WO 2016029566 A
- WO 2016029655 A
- WO 2016029656 A
- WO 2017132852 A
- WO 2017133031 A
- US 20050107764 A1
- US 20120061016 A1
- US 20120061015 A1
- US 7587966 B
- US 447585
- US 13447568
- US 13447544
- US 13447531